Europäisches Patentamt

European Patent Office

Office européen des brevets

(11) Publication number: **0 319 086 B1**

## EUROPEAN PATENT SPECIFICATION

(45) Date of publication of patent specification: **25.03.92**  (51) Int. Cl.⁵: **C07C 57/15**

(21) Application number: **88202672.7**

(22) Date of filing: **24.11.88**

(54) **Pentabasic lead fumarate.**

(30) Priority: **01.12.87 EP 87202374**

(43) Date of publication of application:
**07.06.89 Bulletin 89/23**

(45) Publication of the grant of the patent:
**25.03.92 Bulletin 92/13**

(84) Designated Contracting States:
**AT BE CH DE ES GB IT LI NL**

(56) References cited:
**DE-A- 2 848 566**
**DE-B- 860 209**

(73) Proprietor: **AKZO N.V.**
**Velperweg 76**
**NL-6824 BM Arnhem(NL)**

(72) Inventor: **Frassek, Karl-Heinz**
**Martinusstrasse 16**
**W-5163 Langerwehe(DE)**
Inventor: **Hassa, Klaus**
**Hauptstrasse 156**
**W-4050 Mönchengladbach 2(DE)**

(74) Representative: **Sieders, René et al**
**AKZO N.V. Patent Department (Dept. CO)**
**P.O. Box 9300**
**NL-6800 SB Arnhem(NL)**

EP 0 319 086 B1

Rank Xerox (UK) Business Services

## Description

The invention relates to a new polybasic lead compound and the use thereof as stabilizer for halogen-containing polymers.

Various lead compounds are already known to be used for such a purpose. Monobasic and tetrabasic lead fumarate are known from Swedish Patent Specification 138 952 and Canadian Patent Specification 553 375. In German Offenlegungsschrift 28 48 566 is described a process for preparing a particular lead oxide and the use thereof for the preparation of monobasic up to and including pentabasic lead compounds of inorganic or organic acids, mention also being made of fumaric acid as a suitable organic acid and tetrabasic lead sulphate as a compound having the highest basicity.

Pentabasic lead salicylate, a process for the preparation thereof, and also its application as a pigment are known from U.S. Patent Specifications 2 421 706 and 2 391 166.

The plastics processing industry demands the use of stabilizers which in addition to favourable stabilizing properties have a high degree of whiteness and will also act as pigments in the stabilized plastics.

The present invention has for its object to provide a novel compound displaying an improved stabilizing action as compared with known stabilizers and a higher degree of whiteness.

It has been found that the polybasic lead compound of the total formula x PbO.Pb (trans-COO-CH=CH-COO), wherein x is a number in the range of 4,5-5,8, preferably in the range of 4,7-5,4, shows a surprisingly favourable stabilizing action and has a higher degree of whiteness than the known lead compounds. For simplicity's sake this novel compound will hereinafter be referred to as pentabasic lead fumarate.

The preparation of the pentabasic lead fumarate may take place in the manner commonly used for the preparation of monobasic and polybasic lead compounds.

A customary process for preparing monobasic and polybasic lead compounds is bringing lead oxide into reaction with fumaric acid generally after prereaction with a catalytically active organic acid, e.g. acetic acid, at a temperature in the range of 0°-100°C.

A particularly favourable process of preparation consists in carrying out the catalytic prereaction of lead oxide with an organic acid, for instance formic acid or lead salts of formic acid, preferably at a temperature in the range of 0°-40°C, at which temperature there are subsequently added 7,6-9,4% by weight of fumaric acid calculated on the lead oxide used, the fumaric acid being slowly added all at once or stepwise, preferably in two portions, the temperature during the reaction of the fumaric acid with the lead oxide being increased to 50°-80°C, preferably 55°-70°C.

The pentabasic lead fumarate may additionally be modified to improve its processability in plastics.

Thus, there may be carried out a hydrophobation usually applied in the production of stabilizers, use being made of substances such as silicone oils, fatty acid derivatives, for instance stearic acid, or soft paraffins, which are preferably used in the form of an emulsion, the hydrophobation substances being used in concentrations of 0,5-10% by weight, preferably 2-8% by weight, calculated on pentabasic lead fumarate.

An alternative method for the conversion of the pentabasic lead fumarate is the reaction with organic and/or inorganic acids in a general manner (for instance, as described in US 3 072 693). As organic acids may for instance be used aromatic carboxylic acids (such as toluyl acid, benzoic acid, salicylic acid, phthalic acid, terephthalic acid and derivatives, trimellitic acid, trimesic acid, pyromellitic acid), aliphatic and alicyclic carboxylic acids (for instance, the series of fatty acids such as nonanoic acid, capric acid, undecanoic acid, lauric acid, tridecanoic acid, myristic acid, pentadecanoic acid, palmitic acid, stearic acid, arachidic acid, benic acid up to and including wax acids such as, octacosanic acid, unsaturated higher fatty acids such as oleic acid as well as dicarboxylic acids such as, adipic acid, azelaic acid and sebacic acid, as well as 1,3-cyclopentane carboxylic acid and naphthenic acid), branched fatty acids from the oxo process and neo acids from the Koch process, respectively (such as isooctanoic acid, isononanoic acid, isodecanoic acid, isotridecanoic acid, Versatic acids), which acids may be present as such or in the form of a mixture and are used in concentrations of 10-300% by weight, preferably 10-200% by weight, calculated on the pentabasic lead fumarate.

As examples of suitable inorganic acids may be mentioned sulphuric acid, sulphurous acids, phosphorous acids, phosphoric acid, carbon dioxide, and phosphite-sulphite combinations in concentrations of 4-15% by weight, preferably 5-10% by weight, calculated on the pentabasic lead fumarate, said acids being present as such or mixed.

Furthermore, preferably prior to an optionally additional hydrophobation, such antioxidants may still be added as are commonly used in the plastics industry, e.g. alkyl phenols (such as p-octyl phenol, 4-methyl phenol, 4-butyl phenol, 2,4,6-tri-tert.-butyl phenol, 2,6-di-tert.-butyl-p-cresol, 2,4-dimethyl-6-tert.-butyl phenol, and styrolized phenols) and other sterically hindered phenols, such as alkylidene-diphenols, hydroxybenzyl compounds (such as 2,2'-methylene bis-(4-methyl-6-tert.-butyl phenol), 2,2'-methylene bis-(4-ethyl-6-tert.-butyl phenol), 2,2-bis-(4'-hydroxyphenyl)-propane, tert.-butyl hydroxyanisole, octadecyl-$\beta$-(4'-hydroxy-3',5'-di-tert.-butyl phenol)-propionate, 1,3,5-trimethyl-2,4,6-tri(3,5-di-tert.-butyl-4-hydroxybenzyl)-benzene, 2,2-dihydroxy-3,3'-di-tert.-butyl-5,5'-dimethyl diphenyl methane, 4,4'-butylidene bis-(3-methyl-6-tert.-butylphenol), with the compounds being added in concentrations of 1-12% by weight, preferably 2-6% by weight, calculated on the pentabasic lead fumarate.

Pentabasic lead fumarate shows a strong synergistic action particularly with antioxidants, which manifests itself by the excellent heat stability and resistance to aging of plastics blended with antioxidant-modified pentabasic lead fumarate.

The pentabasic lead fumarate may be added, in modified or unmodified form, to halogen-containing plastics, preferably polyvinyl chloride, polyvinylidene chloride or to their copolymers and blends in concentrations of 0,5-20 parts, preferably 2,5-14 parts, calculated on the polymer.

Alternatively, the pentabasic lead fumarate may be blended with the other substances required for the processing of chlorine-containing polymers such as lubricants, co-stabilizers, fillers, pigments, or combined with them in the melt and subsequently subjected to moulding, to be available in the form of flakes, grit, pellets, or chips as a one-component stabilizer for chlorine-containing polymers.

Pentabasic lead fumarate particularly finds application in the cable industry.

The invention will be further described in, but not limited by, the following examples.

Example 1

In a 2-l flask fitted with a stirrer and pH-electrode 327,8 g of yellow lead monoxide with a density of 9,52 g/cm$^3$ were suspended in 1050 ml of water, which had a temperature of 22°C. To the lead monoxide suspension were added 0,883 ml of 85% formic acid, with continued stirring for 15 minutes. The pH had attained a value of 11,1.

The fumaric acid amount of 28,29 g needed for carrying out the reaction was divided up into two equal portions and one portion in solid form was introduced into the mixture and stirred for 15 minutes at this temperature. The mixture was subsequently heated to 27°C, at which temperature the lead oxide reacted with the fumaric acid, which manifested itself by a thickening of the mixture and evolution of the heat of reaction (T = $\Delta$2°-3°C). The mixture was stirred for 60 minutes at this temperature. Subsequently, the mixture was heated to 60°C within 1 hour, after which the second portion of fumaric acid was introduced. In the following stirring period of 80 minutes the mixture thickened to a degree considerably higher than that in the reaction of the first fumaric acid portion. Simultaneously, the colour of the mixture considerably brightened and changed to white by the end of the stirring period, a pH value of 10,56 being reached.

Subsequently, the solid material was filtered off over a laboratory vacuum filter, sucked dry, dried overnight at 70°C in a laboratory drying cabinet, and ground. Obtained were 350 g of a pentabasic lead fumarate in the form of a white powder having a density of 7,14 g/cm$^3$. The lead content of the product was about 86,4% by weight.

Example 2

Example 1 was repeated and 10,5 g of Bisphenol A were introduced in portions on conclusion of the reaction at 60°C. Each addition of a portion was followed by a reaction, which manifested itself in a thickening of the mixture, which after a short period of stirring disappeared again.

After the total amount of Bisphenol A had been introduced, the mixture was successively stirred for another 15 minutes and filtered, and the solid material was dried and ground as described in Example 1. The yield was about 360 g of a white pentabasic lead fumarate combined with Bisphenol A.

Example 3

In a 5-l flask 730 g of lead oxide were suspended in 2000 ml of water with stirring at a temperature of 20°C. To this lead oxide suspension were added 2,2 g of freshly prepared lead formate, and stirring was continued for 15 minutes. Subsequently, the mixture was heated to 27°C and 28,3 g of fumaric acid were slowly introduced into it. The mixture was stirred for 60 minutes, and subsequently another 28,3 g of fumaric acid were added.

The originally pale yellow reaction mixture gradually turned white. After further stirring for 60 minutes the temperature was increased to 55°C, at which temperature 81 g of solid, powdered stearic acid were added. To keep the mixture stirrable, water was added to it when it became unduly thick. After the treatments in accordance with Example 1 there were obtained 854 g of a white hydrophobated pentabasic lead fumarate having a lead content of 79,0% by weight. This combination has proved outstandingly suitable for use in the cable industry.

Use being made of the product described in this Example, an additional reaction may be carried out with antioxidants.

Example 4

To determine the residual stability of polyvinyl chloride mixed with pentabasic lead fumarate and other stabilizers PVC films were made, and the residual stability determined in accordance with DIN 57 207.
The composition of the films was as follows:

| | |
|---|---|
| PVC, K value 70 (Vinnol H 70 D) | 100 parts |
| trimellitate plasticizer (Reproxal TR 810) | 45 parts |
| calcined china clay (Polystar 501) | 10 parts |
| stabilizer | 7 parts |

As stabilizers were used dibasic lead phthalate, tetrabasic lead fumarate, pentabasic lead salicylate, and pentabasic lead fumarate. The pentabasic lead salicylate was prepared as described in US Patent Specification 2 391 166. The films were made by successively mixing the components and completely gelling them in a laboratory roll mill, with the rolls measuring 115 mm in diameter. Subsequently, 0,35 mm thick film was drawn off. This film was not shaped any further and served to determine the evolution of HCl in accordance with DIN 57 207.

Table I

| Stabilizer | Stability at 200°C determined in accordance with DIN 57 207 [minutes] |
|---|---|
| dibasic lead phthalate | 312 |
| tetrabasic lead fumarate | 368 |
| pentabasic lead salicylate* | 277 |
| pentabasic lead fumarate | 437 |

* pentabasic lead salicylate prepared as described in US Patent Specification 2 391 166

Example 5

Analogous to Example 4 film specimens of 5 x 5 cm were prepared for determining the degree of whiteness. These film specimens were stacked and moulded in a moulding press at 170°C into a sheet measuring 50 x 50 x 5 mm.
The degree of whiteness was measured in a Hunterlab Difference Colorimeter.

Table 2

| Stabilizer | Degree of whiteness L value |
|---|---|
| dibasic lead phthalate | 82,04 |
| tetrabasic lead fumarate | 81,89 |
| pentabasic lead salicylate* | 77,0 |
| pentabasic lead fumarate | 86,43 |

* pentabasic lead salicylate prepared as described in US Patent Specification 2 391 166

4

**Claims**
**Claims for the following Contracting States : AT, BE, CH, DE, GB, IT, LI, NL**

1. A polybasic lead compound of the gross formula

   x PbO.Pb (trans-COO-CH = CH-COO)

   wherein x is a number in the range of 4,5-5,8.

2. A polybasic lead compound according to claim 1, characterized in that the compound is hydrophobated with a hydrophobation agent.

3. A polybasic lead compound according to claim 1, characterized in that the compound is modified with organic and/or inorganic acids.

4. Use of the polybasic lead compound according to any one of the claims 1-3 as stabilizer for halogen-containing polymerizates, copolymerizates and blends.

5. Use according to claim 4, characterized in that polyvinyl chloride or polyvinylidene chloride-containing compositions are stabilized.

6. Use according to any one of claims 4-5, characterized in that the compositions additionally contain antioxidants.

7. Use according to claim 6, characterized in that the antioxidants are sterically hindered phenols.

**Claims for the following Contracting State : ES**

1. A process for the preparation of a polybasic lead compound from lead oxide and fumaric acid, characterized in that the lead oxide and fumaric acid are combined in such amounts that the polybasic lead compound satisfies the following formula:

   x PbO.Pb (trans-COO-CH = CH-COO),

   wherein x is a number in the range of 4,5-5,8.

2. A process according to claim 1, characterized in that the polybasic lead compound is subsequently hydrophobated with a hydrophobation agent

3. A process according to claim 1, characterized in that the polybasic lead compound is subsequently modified with organic and/or inorganic acids.

4. A process for stabilizing halogen-containing polymerizates, copolymerizates and blends, characterized in that a polybasic lead compound obtained from a process according to one any of the claims 1-3 is added.

5. A process according to claim 4, characterized in that polyvinyl chloride or polyvinylidene chloride-containing compositions are stabilized.

6. A process according to any one of the claims 4-5, characterized in that additionally antioxidants are added to the compositions.

7. A process according to claim 6, characterized in that the antioxidants added are sterically hindered phenols.

**Revendications**
**Revendications pour les Etats contractant suivants : AT, BE, CH, DE, GB, IT, LI, NL**

1. Un composé de plomb polybasique de formule générale:

x PbO.Pb (trans-COO-CH = CH-COO)

dans laquelle:
   x est un nombre dans l'intervalle 4,5 - 5,8.

2. Un composé de plomb polybasique selon la revendication 1, caractérisé en ce que ledit composé est rendu hydrophobe par un agent d'hydrophobation.

3. Un composé de plomb polybasique selon la revendication 1, caractérisé en ce que ledit composé est modifié par des acides organiques et/ou inorganiques.

4. Utilisation d'un composé de plomb polybasique selon l'une quelconque des revendications 1 à 3, en tant que stabilisant pour des polymérisats contenant des atomes d'halogène, des copolymérisats et leurs mélanges.

5. Utilisation selon la revendication 4, caractérisée en ce que les compositions contenant du polychlorure de vinylidène ou du polychlorure de vinyle sont stabilisées.

6. Utilisation selon l'une quelconque des revendications 4-5, caractérisée en ce que les compositions contiennent de plus des anti-oxydants.

7. Utilisation selon la revendication 6, caractérisée en ce que les anti-oxydants sont des phénols stériquement bloqués.

**Revendications pour l'Etat contractant suivant : ES**

1. Un procédé de préparation d'un composé de plomb polybasique à partir d'oxyde de plomb et d'acide fumarique, caractérisé en ce que l'oxyde de plomb et l'acide fumarique sont combinés en quantités telles que le composé de plomb polybasique correspond à la formule suivante:

x PbO.Pb (trans-COO-CH = CH-COO)

dans laquelle:
   x est un nombre dans l'intervalle 4,5 - 5,8.

2. Un procédé selon la revendication 1, caractérisé en ce que le composé de plomb polybasique est subséquemment rendu hydrophobe par un agent d'hydrophobation.

3. Un procédé selon la revendication 1, caractérisé en ce que le composé de plomb polybasique est subséquemment modifié par des acides organiques et/ou inorganiques.

4. Un procédé de stabilisation polymérisats et copolymérisats contenant des atomes d'halogène et leurs mélanges, caractérisé en ce qu'un composé de plomb polybasique, obtenu par un procédé selon l'une quelconque des revendications 1 à 3, est ajouté.

5. Un procédé selon la revendication 4, caractérisé en ce que des compositions contenant du polychlorure de vinylidène ou du polychlorure de vinyle sont stabilisés.

6. Un procédé selon l'une quelconque des revendications 4-5, caractérisé en ce que l'on ajoute de plus aux compositions des anti-oxydants.

7. Un procédé selon la revendication 6, caractérisé en ce que les anti-oxydants ajoutés sont des phénols stériquement bloqués.

**Patentansprüche**
**Patentansprüche für folgende Vertragsstaaten : AT, BE, CH, DE, GB, IT, LI, NL**

1. Mehrbasische Bleiverbindung der Summenformel

   x PbO•Pb (trans-COO-CH=CH-COO)

   in der x eine Zahl im Bereich von 4,5 bis 5,8 ist.

2. Mehrbasische Bleiverbindung nach Anspruch 1, dadurch gekennzeichnet, dass die Verbindung mit einem Hydrophobisierungsmittel wasserabstossend gemacht ist.

3. Mehrbasische Bleiverbindung nach Anspruch 1, dadurch gekennzeichnet, dass die Verbindung mit organischen und/oder anorganischen Säuren modifiziert ist.

4. Verwendung der mehrbasischen Bleiverbindung nach einem der Ansprüche 1-3 als Stabilisator für Halogen enthaltende Polymerisate, Copolymerisate und Mischungen.

5. Verwendung nach Anspruch 4, dadurch gekennzeichnet, dass Polyvinylchlorid oder Polyvinylidenchlorid enthaltende Zubereitungen stabilisiert werden.

6. Verwendung nach einem der Ansprüche 4-5, dadurch gekennzeichnet, dass die Zubereitungen ausserdem Antioxidationsmittel enthalten.

7. Verwendung nach Anspruch 6, dadurch gekennzeichnet, dass die Antioxidationsmittel sterisch behinderte Phenole sind.

**Patentansprüche für folgenden Vertragsstaat : ES**

1. Verfahren zur Herstellung mehrbasischer Bleiverbindungen aus Bleioxid und Fumarsäure, dadurch gekennzeichnet, dass das Bleioxid und die Fumarsäure in solchen Anteilen zusammengebracht werden, dass die mehrbasische Bleiverbindung die folgende Formel erfüllt:

   x PbO•Pb (trans-COO-CH=COO)

   in der x eine Zahl im Bereich von 4,5 bis 5,8 ist.

2. Verfahren nach Anspruch 1, dadurch gekennzeichnet, dass die mehrbasische Bleiverbindung nachfolgend mit einem Hydrophobisierungsmittel wasserabstossend gemacht wird.

3. Verfahren nach Anspruch 1, dadurch gekennzeichnet, dass die mehrbasische Bleiverbindung nachfolgend mit organischen und/oder anorganischen Säuren modifiziert wird.

4. Verfahren zum Stabilisieren von Halogen enthaltenden Polymerisaten, Copolymerisaten und Mischungen, dadurch gekennzeichnet, dass eine mehrbasische Bleiverbindung zugegeben wird, die nach einem Verfahren gemäss einem der Ansprüche 1-3 erhalten worden ist.

5. Verfahren nach Anspruch 4, dadurch gekennzeichnet, dass Polyvinylchlorid oder Polyvinylidenchlorid enthaltende Zubereitungen stabilisiert werden.

6. Verfahren nach einem der Ansprüche 4-5, dadurch gekennzeichnet, dass zu den Zubereitungen ausserdem Antioxidationsmittel gegeben werden.

7. Verfahren nach Anspruch 6, dadurch gekennzeichnet, dass die zugesetzten Antioxidationsmittel sterisch behinderte Phenole sind.

7